**Europäisches Patentamt**

**European Patent Office**

**Office européen des brevets**

(19)

(11) Veröffentlichungsnummer: **0 271 815 B1**

# EUROPÄISCHE PATENTSCHRIFT

(12)

(45) Veröffentlichungstag der Patentschrift: **20.03.91**

(51) Int. Cl.5: **C07D 201/08**

(21) Anmeldenummer: **87118199.6**

(22) Anmeldetag: **09.12.87**

(54) Verfahren zur Herstellung von Caprolactam.

(30) Priorität: **17.12.86 DE 3643010**

(43) Veröffentlichungstag der Anmeldung:
**22.06.88 Patentblatt 88/25**

(45) Bekanntmachung des Hinweises auf die
Patenterteilung:
**20.03.91 Patentblatt 91/12**

(84) Benannte Vertragsstaaten:
**BE CH DE ES FR GB IT LI NL**

(56) Entgegenhaltungen:
**GB-A- 2 108 119**

(73) Patentinhaber: **BASF Aktiengesellschaft
Carl-Bosch-Strasse 38
W-6700 Ludwigshafen(DE)**

(72) Erfinder: **Merger, Franz, Dr.
Max-Slevogt-Strasse 25
W-6710 Frankenthal(DE)**
Erfinder: **Priester, Claus-Ulrich, Dr.
Wiesenstrasse 18
W-6701 Meckenheim(DE)**
Erfinder: **Fischer, Rolf, Dr.
Bergstrasse 98
W-6900 Heidelberg(DE)**
Erfinder: **Sauerwald, Manfred, Dr.
Gebhardtstrasse 16
W-6701 Roedersheim-Gronau(DE)**

EP 0 271 815 B1

## Beschreibung

Gegenstand der Erfindung ist ein Verfahren zur Herstellung von Caprolactam aus 6-Aminocapronsäureestern.

In der DE-OS 22 49 993 wird ein Verfahren beschrieben, bei dem man 6-Aminocapronsäureester in Gegenwart von Wasser bei einer Temperatur von 250 bis 350°C zu Caprolactam umsetzt.

Das Verfahren hat jedoch den Nachteil, daß, wie aus Industrial Engineering Chem. Proc. Design Development, Band 17 (1978), Seite 11, bekannt, diese Umsetzung über 6-Aminocapronsäure verläuft und daher, wie aus Seite 15 ersichtlich, die Cyclisierung bei niedrigen Konzentrationen durchgeführt werden soll, um die Bildung von Oligomeren zu unterdrücken. Dies hat zur Folge, daß sich die Isolierung des Caprolactams aus den verdünnten wäßrigen Lösungen technisch aufwendig gestaltet.

Aus Industrial Engineering Chem. Proc. Design Development, Band 17 (1978), Seiten 10 und 11, ist auch schon die Cyclisierung von 6-Aminocapronsäureethylester in Ethanol bei einer Temperatur von 150 bis 250°C zu Caprolactam bekannt. Hierbei werden jedoch erhebliche Mengen an Dimeren als Nebenprodukte gebildet, wodurch die Ausbeute zu wünschen übrig läßt.

Nach einem anderen in der DE-OS 32 35 938 beschriebenen Verfahren werden 6-Aminocapronsäureester durch Erhitzen auf 180 bis 250°C in einem mehrwertigen Alkohol mit einem Siedepunkt, der höher ist als derjenige des Caprolactams, in einem Zeitraum von 0,5 bis 5 Stunden zu Caprolactam umgesetzt und anschließend Caprolactam aus dem erhaltenen Reaktionsgemisch durch Destillation abgetrennt. Dieses Verfahren hat den Nachteil, daß anspruchsvolle Lösungsmittel wie Tetraethylenglykol, Diglycerin, Pentaerythrit oder Butantriol angewandt werden, die thermolabil sind. Zudem ist die Umsetzung nicht selektiv, sondern es bilden sich Nebenprodukte ("Aminocaprolactam-Umwandlungsprodukte") und Caprolactam-Oligomere, die zurückgeführt werden müssen.

Aus der japanischen Patentveröffentlichung 14563/1963 ist auch schon ein Verfahren bekannt, bei dem man 6-Aminocapronsäureethylester bei einer Temperatur von 160 bis 165°C innerhalb von 4 Stunden in siedendem Ethylenglykol zu Caprolactam kondensiert. Dieses Verfahren hat den Nachteil, daß die Ausbeute zu wünschen übrig läßt und sich zudem die Gewinnung des Caprolactams aus den Reaktionsmedium technisch aufwenig gestaltet.

Es was deshalb die technische Aufgabe gestellt, ein Verfahren zur Herstellung von Caprolactam, ausgehend von 6-Aminocapronsäureestern, zur Verfügung zu stellen, das mit hohen Ausbeuten und hohen Umsätzen in kurzer Zeit verläuft, keine aufwenaigen Lösungsmittel erfordert, eine einfache Abtrennung des Caprolactams ermöglicht und bei dem Rückführungen von Nebenprodukten und Abscheidungen durch Bildung von Polymerisaten des Caprolactams vermieden werden.

Diese technische Aufgabe wird gelöst in einem Verfahren zur Herstellung von Caprolactam durch Erhitzen von 6-Aminocapronsäureestern von Alkanolen mit 1 bis 10 Kohlenstoffatomen in Gegenwart von einem unter Reaktionsbedingungen flüssigen inerten organischen Reaktionsmedium mit einem Siedepunkt unterhalb dem des Caprolactams, wobei man als Reaktionsmedium aromatische Kohlenwasserstoffe mit einem Siedepunkt von 80 bis 240°C verwendet, eine Temperatur von 100 bis 320°C einhält und aus dem Reaktionsmedium Caprolactam gewinnt.

Das neue Verfahren hat den Vorteil, daß es mit hohem Umsatz und hohen Ausbeuten verläuft. Ein weiterer Vorteil des Verfahrens ist es, daß wenig aufwendige Lösungsmittel verwendet werden und die Gewinnung des Caprolactams einfach gestaltet wird. Weiter hat das neue Verfahren den Vorteil, daß weder Produkte gebildet werden, die rückgeführt werden müssen, noch in deutlichem Maße Polymerisate, die zu Ablagerungen führen.

Die Cyclokondensation von 6-Aminocapronsäureestern in Kohlenwasserstoffen ist bisher nicht beschrieben. Zwar erwähnt man in DE-OS 32 35 938 die Möglichkeit des Einsatzes von Kohlenwasserstoffen mit einem Siedepunkt oberhalb dem des Caprolactams, verwirft sie aber mit der Anmerkung, daß ein unlösliches Polymerisat entstehe. Vor der Verwendung eines Lösungsmittels mit einem Siedepunkt unterhalb dem des Caprolactams warnt man im gleichen Zusammenhang mit dem Hinweis, es entstünden Nebenprodukte, die bei der destillativen Aufarbeitung denaturieren und daher unbrauchbar seien.

Als Ausgangsstoffe verwendet man 6-Aminocapronsäureester von Alkanolen mit 1 bis 10 Kohlenstoffatomen. Geeignete Ausgangsstoffe sind beispielsweise 6-Aminocapronsäuremethylester, -ethylester, -n-propylester oder -n-butylester. Besonders bevorzugt sind 6-Aminocapronsäure-$C_1$-$C_3$alkylester. Besondere technische Bedeutung haben 6-Aminocapronsäuremethyl-oder -ethylester erlangt.

Die Umsetzung wird bei einer Temperatur von 100 bis 320°C, vorteilhaft 130 bis 280°C, insbesondere 180 bis 280°C, durchgeführt. Im allgemeinen hält man bei der Umsetzung Atmosphärendruck ein, bevorzugt wendet man jedoch erhöhten Druck an, z.B. bis zu 30 bar, um sicherzustellen, daß das Reaktionsmedium in flüssiger Phase vorliegt.

Erfindungsgemäß wird die Umsetzung in Gegenwart von unter Reaktionsbedingungen flüssigen aromatischen Kohlenwasserstoffen mit einem Siedepunkt von 80 bis 240°C, insbesondere mit einem Siedepunkt von 110 bis 200°C durchgeführt. Bevorzugte aromatische Kohlenwasserstoffe sind Alkylbenzole, insbesondere solche, die 1 bis 2 Alkylgruppen mit bis zu 6 Kohlenstoffatomen enthalten. Besonders bevorzugt sind Alkylbenzole mit 1 bis 3 Alkylresten mit insgesamt bis zu 4 Kohlenstoffatomen. Geeignete aromatische Kohlenwasserstoffe sind beispielsweise Benzol, Toluol, Xylole, Ethylbenzol, Diethylbenzol, Trimethylbenzol, Isopropylbenzol, Propylbenzol oder Diisopropylbenzol.

Die verwendeten 6-Aminocapronsäuren können sowohl in reiner Form als auch in Form einer Lösung, z.B. in Alkanolen oder dem als Reaktionsmedium verwendeten aromatischen Kohlenwasserstoff, in die Reaktion eingeführt werden. Falls Alkanole als Lösungsmittel verwendet werden, ist es zweckmäßig, die Alkanole in gleichem Maß aus dem System zu entfernen (z.B. durch Destillation), wie alkoholische Lösung von 6-Aminocapronsäureester zugeführt wird, so daß eine Anreicherung des Alkohols verhindert wird.

Zweckmäßigerweise entsprechen die Alkanole der Alkoholkomponente des verwendeten 6-Aminocapronsäureesters, z.B. 6-Aminocapronsäuremethylester in Methanol. Geeignete Lösungen haben einen Gehalt von 5 bis 25 Gew.-% 6-Aminocapronsäureester.

Vorteilhaft wendet man je kg 6-Aminocapronsäureester 2 bis 20 kg aromatische Kohlenwasserstoffe als Reaktionsmedium an.

Vorzugsweise hält man eine Verweilzeit von 0,25 bis 15 Stunden ein.

Die Umsetzung wird beispielsweise so durchgeführt, daß man 6-Aminocapronsäureester, vorzugsweise unter guter Durchmischung, in aromatischen Kohlenwasserstoffen auf die angegebene Temperatur erhitzt, wobei man die Druck- und Temperaturbedingungen so wählt, daß das Reaktionsmedium stets in flüssigem Zustand vorliegt. Vorteilhaft werden hierbei die abgespaltenen Alkohole fortlaufend aus dem Reaktionsgemisch, z.B. durch Destillation, entfernt. Nach einer anderen Arbeitsweise wird die Umsetung beispielsweise so durchgeführt, daß man eine Lösung von 6-Aminocapronsäureester in Alkanolen bei der angegebenen Temperatur kontinuierlich in die aromatischen Kohlenwasserstoffe einbringt, bis die gewünschte Konzentration von 6-Aminocapronester und Caprolactam, beispielsweise 5 bis 25 %, erreicht ist und gleichzeitig das als Lösungsmittel eingesetzte und bei der Cyclisierung gebildete Alkanol durch Destillation abtrennt. Die Reaktion wird dann durch weiteres Erhitzen auf die angegebene Temperatur vervollständigt und dabei zweckmäßigerweise der bei der Cyclisierung zu Caprolactam freigesetzte Alkohol durch Destillation entfernt.

Nach einer anderen bevorzugten Arbeitsweise wird eine Lösung von 6-Aminocapronsäureester in dem als Reaktionsmedium verwendeten aromatischen Kohlenwasserstoff kontinuierlich einer Kaskade aus mehreren Reaktionsbehältern, z.B. 2 bis 4 Reaktionsbehältern, zugeführt, gegebenenfalls freigesetzte Alkanole fortwährend abgetrennt und im letzten Reaktionsbehälter eine Lösung von Caprolactam in dem Maße entnommen, wie Ausgangslösung zugeführt wird. Bei einer anderen Arbeitsweise werden die Alkanole nach vollendeter Umsetzung, z.B. durch Entspannen, aus dem Reaktionsgemisch entfernt. Diese Arbeitsweise eignet sich insbesondere, wenn man 6-Aminocapronsäureester enthaltende aromatische Kohlenwasserstoffe kontinuierlich einer langgestreckten Reaktionszone, z.B. einem Rohrreaktor oder Rohrschlange zuführt, die Umsetzung bei den oben angegebenen Bedingungen durchführt und nach Entnahme des Reaktionsgemisches, z.B. durch Entspannen oder Destillation, Alkohole abtrennt.

Aus der so erhaltenen Lösung von Caprolactam in aromatischen Kohlenwasserstoffen wird Caprolactam im allgemeinen durch fraktionierende Destillation gewonnen und die aromatischen Kohlenwasserstoffe wieder zurückgeführt. Nach einer bevorzugten Arbeitsweise wird Caprolactam aus den aromatischen Kohlenwasserstoffen mit Wasser extrahiert. Vorteilhaft wird die Extraktion im Gegenstrom in bekannten Vorrichtungen, z.B. Mixer Settler, Rührscheibenkolonnen oder Siebbodenkolonnen mit oder ohne Pulsation durchgeführt. Vorteilhaft hält man bei der Extraktion eine Temperatur von 20 bis 80°C ein. (Gegebenenfalls wird ein Teilstrom der aromatischen Kohlenwasserstoffe vor der Wiederverwendung durch Destillation gereinigt.)

Das Verfahren nach der Erfindung sei an folgenden Beispielen veranschaulicht.

Beispiel 1

Eine Lösung von 95,9 g (0,661 mol) 6-Aminocapronsäuremethylester in 1500 g o-Xylol wird 14 Stunden bei 140°C erhitzt. Das gebildete Methanol wird kontinuierlich durch Destillation entfernt. Nach Abdestillieren des Xylols in Vakuum verbleiben 74.6 g (0,660 mol) Caprolactam; Caprolactam-Ausbeute: 99,8 %.

Beispiel 2

Eine Lösung von 80,4 g (0,555 mol) 6-Aminocapronsäuremethylester in 900 g Toluol wird 9 h bei 110°C unter kontinuierlichem Abdestillieren ei-·

nes Methanol-Toluol-Gemisches erhitzt. Nach Abdampfen des Toluols in Vakuum und Reinigung durch Sambay-Destillation bei 0,3 mbar und 120 bis 135°C verbleiben 56,8 g (0,503 mol); Ausbeute: 90,6 %) Caprolactam.

Beispiel 3

Eine Lösung von 11,4 g (0,078 mol) 6-Aminocapronsäuremethylester in 118 g Methanol wird innerhalb von 4,5 h bei 130°C und 4,0 bar in einen mit 279 g Benzol gefüllten 250-ml-Glasautoklaven zugepumpt. 213 g eines Methanol-Benzol-Gemisches werden in dem Maße aus dem System durch Destillation entfernt, wie methanolische 6-Aminocapronsäuremethylester-Lösung zugeführt und Methanol bei der Cyclisierung zu Caprolactam gebildet wird. Nach einer weiteren Stunde bei 130°C und Abdampfen des Benzols in Vakuum verbleiben 8,6 g (0,076 mol; Ausbeute: 97,4 %) Caprolactam.

Beispiel 4

Eine Lösung von 73,9 g (0,510 mol) 6-Aminocapronsäuremethylester in 1000 g Ethylbenzol wird 10 h bei 138°C unter Rückfluß erhitzt. Bei der Reaktion gebildetes Methanol wird durch kontinuierliche Destillation entfernt. Nach Extraktion der Reaktionslösung mit Wasser und einrotieren der wäßrigen Phase in Vakuum werden 54,0 g (0,478 mol; Ausbeute: 93,8 %) Caprolactam erhalten.

Beispiel 5

65,3 g (0,450 mol) 6-Aminocapronsäuremethylester werden in 1000 g Cumol gelöst und 10 h bei 150°C erhitzt. Gebildetes Methanol wird kontinuierlich durch Destillation entfernt. Nach Abdampfen des Cumols in Vakuum werden 48,5 g (0,429 mol; Ausbeute: 95,3 %) Caprolactam isoliert.

Beispiel 6

Eine Lösung von 37,1 g (0,256 mol) 6-Aminocapronsäuremethylester in 723 g Mesitylen wird 12 h bei einem Druck von 500 bar und 140°C unter Rückfluß gerührt. Nach Abdampfen des Mesitylens unter Vakuum verbleiben 46,4 g eines Caprolactam-Mesitylen-Gemisches, das nach HPLC 58,2 % Caprolactam enthält, entsprechend 27,0 g (0,239 mol) und einer Ausbeute von 93,4 %.

Beispiel 7

In eine erste Stufe (Stufe 1: Volumen 800 ml; Stufe 2: Volumen 1200 ml) werden stündlich 227 g Xylol und 200 g einer methanolischen Lösung von 19,2 g (0,132 mol) 6-Aminocapronsäuremethylester zugepumpt und auf 140°C erhitzt. Stündlich werden 211 g eines Methanol-Xylol-Gemisches aus Stufe 1 über eine 37-cm-Füllkörperkolonne abdestilliert. Die so erhaltene Lösung wird kontinuierlich einer zweiten Stufe zugeführt, auf 140°C gehalten und 215,9 g einer xylolischen Caprolactam-Lösung aus Stufe 2 ausgetragen. Nach Abdestillieren des Xylols verbleiben 13,6 g (0,120 mol) Caprolactam und 0,8 g (0,005 mol) 6-Aminocapronsäuremethylester pro Stunde entsprechend einem Umsatz von 96 % und einer Selektivität von 94,5 %.

Beispiel 8

In eine Dreierkaskade (Stufe 1: Volumen 800 ml, Stufe 2: Volumen 1200 ml, Stufe 3: Volumen 600 ml) werden stündlich 227 g Xylol und 200 g einer methanolischen Lösung von 19,2 g (0,132 mol) 6-Aminocapronsäuremethylester zugepumpt und auf 140°C erhitzt. Stündlich werden 211 g eines Methanol-Xylol-Gemisches aus Stufe 1 über eine 37-cm-Füllkörperkolonne abdestilliert. Die so erhalten Lösung wird durch zwei weitere Stufen bei 140°C geleitet und 215,9 g einer xylolischen Caprolactam-Lösung aus Stufe 3 ausgetragen. Nach Abdestillieren des Xylols verbleiben 14,4 g (0,127 mol) Caprolactam und 0,3 g (0,002 mol) 6-Aminocapronsäuremethylester pro Stunde, entsprechend einem Umsatz von 98,4 % und einer Selektivität von 97,7 %.

Beispiel 9

In einen Rührreaktor mit einem Volumen von 800 ml werden stündlich 230 g Xylol und 205 g einer methanolischen Lösung von 18,5 g (0,128 mol) 6-Aminocapronsäuremethylester zugepumpt und auf 140°C erhitzt. Stündlich werden 216,4 g eines Methanol-Xylol-Gemisches über eine 37-cm-Füllkörperkolonne abdestilliert und 218,7 g einer xylolischen Caprolactam-Lösung ausgetragen. Diese wird bei 270°C, 27 bar und einer mittleren Verweilzeit von 45 min durch einen Rohrreaktor gepumpt. Nach Abdestillieren des Xylols verbleiben 13,8 g (0,122 mol) Caprolactam/Stunde, entsprechend einer Ausbeute von 95,3 %.

Beispiel 10

Eine Lösung von 45,1 g (0,311 mol) 6-Aminocapronsäuremethylester und 19,0 g (0,168 mol) Caprolactam in 700 g o-Xylol wird bei 270°C, einem Druck von 27 bar und einer mittleren Verweilzeit von 1,0 h durch einen Rohrreaktor gepumpt. Nach Abdestillieren des Xylols und Methanols in Vakuum erhält man 0,8 g (0,006 mol) 6-Aminocapronsäuremethylester und 52,2 g(0,462 mol) Caprolactam, entsprechend einem Umsatz von 98,1 % und einer Selektivität von 96,4 %.

Vergleichsbeispiel 1 (B510/1)

(Stabilität von Caprolactam in Polyethylenglykol)

Eine Lösung von 20 g Caprolactam in 180 g Polyethylenglykol (HO-(CH$_2$-CH$_2$-O)n-H; mittleres Molekulargewicht: 400) wird bei 190°C gerührt. Nach 4 h hat der Caprolactam-Gehalt (überprüft durch HPLC-Analytik) auf 19,7 % abgenommen, nach 7 h auf 18,5 %. Eine Lösung von 10 g Caprolactam in 190 g Polyethylenglykol (mittleres Molekulargewicht: 400) wird bei 190°C gerührt. Nach 4 h hat der Caprolactam-Gehalt (überprüft durch HPLC-Analytik) auf 8,6 % abgenommen, nach 7 h auf 7,9 %.

Vergleichsbeispiel 2

Eine Lösung von 63 g (0,434 mol) 6-Aminocapronsäuremethylester und 33 g (0,292 mol) Caprolactam in 300 g Wasser wird bei einer mittleren Verweilzeit von 4 Minuten bei einer Temperatur von 275°C durch einen 40 ml-Rohrreaktor gepumpt. Die Reaktionslösung enthält nach quant. GC 15 % Caprolactam und als Nebenprodukt 1 % N-Methyl-Caprolactam. Die Caprolactam-Selektivität beträgt bei einem 6-Aminocapronsäuremethylesterumsatz von 100 % 54 %.

Vergleichsbeispiel 3

Eine Lösung von 85,6 g (0,590 mol) 6-Aminocapronsäuremethylester in 900 g Dekalin wird 8 h bei 150°C zum Sieden erhitzt. Bei der Cyclisierung gebildetes Methanol wird kontinuierlich durch Destillation entfernt. Während der Reaktion fallen 2,6 g Polymere aus. Abdampfen des Dekalins in Vakuum und Sambay-Destillation bei 0,3 mbar und 120 bis 135°C liefert 49,6 g (0,439 mol; Ausbeute: 74,4 %) Caprolactam.

Vergleichsbeispiel 4

Eine Lösung von 110,5 g (0,762 mol) 6-Aminocapronsäuremethylester in 1032 g Dodecylbenzol (KP 280°C ) wird unter kontinuierlichem Abdestillieren des gebildeten Methanols 4,5 h bei 180°C gerührt. Nach Extraktion des Dodecylbenzols mit Wasser und Abdestillieren des Wassers in Vakuum verbleiben 76,3 g (0,675 mol; Ausbeute: 88,6 %) Caprolactam.

Ansprüche

1. Verfahren zur Herstellung von Caprolactam durch Erhitzen von 6-Aminocapronsäureestern von Alkanolen mit 1 bis 10 Kohlenstoffatomen in Gegenwart von einem unter Reaktionsbedingungen flüssigen inerten organischen Reaktionsmedium mit einem Siedepunkt unterhalb dem des Caprolactams, dadurch gekennzeichnet, daß man als Reaktionsmedium aromatische Kohlenwassertoffe mit einme Siedepunkt von 80 bis 240°C verwendet, eine Temperatur von 100 bis 320°C einhält und aus dem Reaktionsmedium Caprolactam gewinnt.

2. Verfahren nach Anspruch 1, dadurch gekennzeichnet, daß man Alkylbenzole mit einem Siedepunkt von 110 bis 200°C als Reaktionsmedium verwendet.

3. Verfahren nach den Ansprüchen 1 und 2, dadurch gekennzeichnet, daß man Alkylbenzole mit 1 bis 3 Alkylresten mit insgesamt bis zu 4 Kohlenstoffatomen verwendet.

4. Verfahren nach den Ansprüchen 1 bis 3, dadurch gekennzeichnet, daß man eine Temperatur von 130 bis 280°C einhält.

5. Verfahren nach den Ansprüchen 1 bis 4, dadurch gekennzeichnet, daß man die als Spaltprodukte anfallenden Alkohole während der Umsetzung durch Destillation abtrennt.

6. Verfahren nach den Ansprüchen 1 bis 5, dadurch gekennzeichnet, daß man Caprolactam aus den aromatischen Kohlenwasserstoffen durch Extraktion mit Wasser abtrennt.

7. Verfahren nach den Ansprüchen 1 bis 7, dadurch gekennzeichnet, daß man 6-Aminocapronsäuremethyl- oder -ethylester als Ausgangsverbindungen verwendet.

Claims

1. A process for preparing caprolactam by heating a 6-aminocaproic ester of an alkanol of 1 to 10 carbon atoms in the presence of an inert organic reaction medium which is liquid under the reaction conditions and has a boiling point below that of caprolactam, which comprises using as the reaction medium an aromatic hydrocarbon having a boiling point of from 80 to 240°C, maintaining a temperature of from 100 to 320°C, and isolating the caprolactam from the reaction medium.

2. A process as claimed in claim 1, wherein the reaction medium used is an alkylbenzene having a boiling point from 110 to 200°C.

3. A process as claimed in claim 1 or 2, wherein an alkylbenzene having 1 to 3 alkyl radicals of up to 4 carbon atoms in total is used.

4. A process as claimed in any of claims 1 to 3, wherein a temperature from 130 to 280°C is maintained.

5. A process as claimed in any of claims 1 to 4, wherein the alcohol obtained as an elimination product is separated off by distillation in the course of the reaction.

6. A process as claimed in any of claims 1 to 5, wherein the caprolactam is separated from the aromatic hydrocarbon by extraction with water.

7. A process as claimed in any of claims 1 to 6, wherein the starting compound used is methyl 6-aminocaproate or ethyl 6-aminocaproate.

**Revendications**

1. Procédé de préparation de caprolactame par cnauffage d'esters d'acide 6-aminocaproïque et d'alcanols à 1-10 atomes de carbone en présence d'un milieu réactionnel organique inerte, liquide dans les conditions de la réaction et ayant un point d'ébullition inférieur à celui du caprolactame, caractérisé en ce qu'on utilise comme milieu réactionnel des hydrocarbures aromatiques ayant un point d'ébullition entre 80 et 240°C, on maintient une temperature de 100 a 320°C et on extrait le caprolactame du milieu réactionnel.

2. Procédé selon la revendication 1, caractérisé en ce qu'on utilise comme milieu réactionnel des alkylbenènes ayant un point d'ébullition entre 110 et 200°C.

3. Procédé selon la revendication 1 ou 2, caractérisé en ce qu on utilise des alkylbenzènes renfermant de 1 à 3 restes alkyle avec, au total, jusqu'à 4 atomes de carbone.

4. Procédé selon l'une quelconque des revendications 1 à 3, caractérisé en ce qu'on maintient une température de 130 à 280°C.

5. Procédé selon l'une quelconque des revendications 1 à 4, caractérisé en ce qu'au cours de la réaction, on sépare par distillation les alcools qui se forment en tant que produits de coupure.

6. Procédé selon l'une quelconque des revendications 1 à 5, caractérisé en ce qu'on sépare le caprolactame des hydrocarbures aromatiques par extraction à l'eau.

7. Procédé selon l'une quelconque des revendications 1 à 7. caractérisé en ce qu'on utilise, comme composé de départ, l'ester méthylique ou éthylique d'acide 6-aminocaproïque.